# EUROPÄISCHE PATENTSCHRIFT

(11) **EP 2 829 242 B1**
(45) Veröffentlichungstag und Bekanntmachung des Hinweises auf die Patenterteilung: **31.01.2018**
(21) Anmeldenummer: 14177888.6
(22) Anmeldetag: 21.07.2014
(51) Int. Cl.: A61B 17/70, A61B 17/68, A61B 17/86, A61B 17/80, A61L 31/06, A61L 31/08, A61L 31/14

(54) **System zur Verankerung einer Pedikelschraube, zugehöriger Bausatz, Spacer und Pedikelaugmentat**
System for anchoring a pedicle screw, associated kit, spacer and pedicle graft
Système d'ancrage d'une vis pédiculaire, élément associé, espaceur et matière d'augmentation pédiculaire

(30) Priorität: 23.07.2013 DE 102013107881
(43) Veröffentlichungstag der Anmeldung: 28.01.2015
(73) Patentinhaber: AESCULAP AG, 78532 Tuttlingen (DE)
(72) Erfinder: Beger, Jens, 78532 Tuttlingen (DE); Klingseis, Susanne, 88400 Biberach (DE)
(74) Vertreter: Winter, Brandl, Fürniss, Hübner, Röss, Kaiser, Polte - Partnerschaft mbB

(56) Entgegenhaltungen:
- WO-A1-2008/129241
- WO-A1-2010/019979
- US-A1- 2008 221 623
- US-A1- 2010 145 463
- US-A1- 2011 087 296
- US-A1- 2012 271 309
- US-A1- 2013 035 724

## Beschreibung

Die Erfindung betrifft ein System zur Verankerung einer Pedikelschraube in einem Wirbel eines Patienten, mit einer Pedikelschraube, die einen im Wirbelbogen einschließlich des Pedikels versenkbaren Gewindeabschnitt aufweist, und zumindest einem den Raum zwischen Gewindeabschnitt und kortikaler Randschicht des Pedikels zumindest bereichsweise ausfüllenden Spacer. Bei derartigen Systemen wird eine Knochenschraube, wie zum Beispiel eine Pedikelschraube in den Wirbelbogen und in den sich daran anschließenden Pedikel eingeschraubt, wobei die Verankerung über eine besondere Gestaltung des Pedikelschraubengewindes und/oder des Kernquerschnitts der Pedikelschraube gesteuert wird.

Pedikelschrauben werden in erster Linie für ein posteriores thorakolumbales Stabilisations-System verwendet, bei dem mit einem Schrauben-Stab-System eine Wirbelsäulenfixierung angestrebt wird. Die dabei eingesetzten Pedikelschrauben sind erheblichen Beanspruchungen ausgesetzt, sodass verschiedenste Ansätze bekannt geworden sind, mit denen die Verankerung im Pedikelkanal verbessert werden sollte.

So ist beispielsweise aus dem Dokument US 8,388,660 B1 eine Anordnung bekannt, bei der sich ein Schraubenschaft durch eine Gewindehülse erstreckt, die zur Verankerung im Pedikel im Übergang zum Wirbelkörper aufgespreizt wird.

Ein System gemäß dem Oberbegriff des Anspruchs 1 ist aus dem Dokument US 2008/221623 A1 bekannt. Dabei findet als Spacer eine Art Dübel Anwendung, dessen spreizbare Abschnitte beim Einschrauben der Pedikelschraube konzentrisch aufgespreizt werden.

Im Dokument US 2012/0116465 A1 wird ebenfalls eine Schraube verwendet, die einen sich radial aufspreizbaren distalen Gewindeabschnitt hat.

Gemäß US 8,343,200 B2 wird eine Pedikelschraube verwendet, die in eine Schraubenhülse eingedreht wird. Gemäß US 7,935,138 B1 wird bei einer ähnlichen Konstruktion die äußere Schraubenhülse geschlitzt ausgeführt.

Schließlich ist aus dem Dokument US 2008/0086131 A1 ein System bekannt geworden, bei dem die zusätzliche Fixierung der Pedikelschraube durch einen unterhalb des Schraubenkopfs liegenden Ring erfolgt, der sich mit dornenartigen Fortsätzen in den Wirbelbogen eingräbt.

Alle bekannten Lösungen haben entweder den Nachteil, dass das radiale Aufspreizen mit zu geringer Kontrolle erfolgt, wodurch sich eine erhöhte Frakturgefahr ergibt, oder aber den Nachteil (bei Verwendung der Spikes gemäß US 2008/0086131 A1) einer mehr oder weniger unkontrollierten Einwirkung auf die Knochensubstanz, was zu deren Schädigung führen kann.

Der Erfindung liegt deshalb die Aufgabe zugrunde, ein System zur Verankerung einer Pedikelschraube in einem Wirbel eines Patienten, einen zugehörigen Bausatz für dieses System und die dazu erforderlichen Komponenten zu schaffen, mit denen es gelingt, die Verankerungsstabilität unter möglichst guter Schonung der Knochensubstanz anzuheben.

Diese Aufgabe wird durch die Merkmale des Patentanspruchs 1 gelöst.

Erfindungsgemäß wird der Weg einer Pedikelaugmentation eingeschlagen, um eine Rundum-Abstützung der Pedikelschraube an der sogenannten Kortikalis, das heißt an der kortikalen Randschicht des Wirbelbogens einschließlich der Wirbelbogenwurzel (Pedikel) zu erreichen. Die Erfindung beruht dabei auf folgenden Überlegungen:
Der Pedikel eines lumbalen und thorakalen Wirbels beschreibt annäherungsweise eine ovale Struktur, die durch die äußere kortikale Schicht, der sogenannten Compacta, und einer spongiösen inneren Struktur, der sogenannten Spongiosa, gebildet wird. Üblicherweise kann sich dabei eine zylindrische Pedikelschraube nur an zwei Seiten der Pedikelwand abstützen. Grundsätzlich ist es möglich, die Schraube möglichst cranial oder caudal zu positionieren, wodurch ein weiterer Abstützpunkt bereitgestellt werden kann. Damit ist jedoch noch nicht das Problem gelöst, die Querkräfte in cranio-caudaler Richtung und die in dieser Ebene auftretenden Biegemomente zuverlässig aufzunehmen, damit es zu keinem sogenannten "Scheibenwischereffekt", das heißt zum Auslockern der Pedikelschraube kommt.

Erfindungsgemäß wird hingegen ein den Raum zwischen Gewindeabschnitt und kortikaler Randschicht des Pedikels zumindest bereichsweise ausfüllender Spacer verwendet, der so gestaltet bzw. bezüglich des Gewindeabschnitts im Pedikelquerschnitt positioniert ist, dass über den Spacer - es können auch mehrere Spacer zum Einsatz kommen - eine Rundum-Abstützung des Gewindeabschnitts der Pedikelschraube an der kortikalen Randschicht ermöglicht ist. Diese Rundum-Abstützung erlaubt es, die oben angesprochenen Querkräfte in cranio-caudaler Richtung und die bei der Wirbelfixierung auf die Pedikelschrauben einwirkenden Biegemomente wesentlich sicherer aufzunehmen, als dies mit herkömmlichen Systemen der Fall ist, was zu einer erheblichen Verbesserung der Operationstechnik führt.

Die Realisierung der Pedikelaugmentation kann auf verschiedene Art und Weise erfolgen.

Gemäß einer ersten Variante, die nicht Gegenstand der vorliegenden Erfindung ist, wird ein den Pedikelquerschnitt bis zur kortikalen Randschicht ausfüllender zylindrischer Körper verwendet, der formschlüssig in einer entsprechenden, in den Pedikel eingearbeiteten axialen Ausnehmung des Pedikels sitzt und eine axiale Durchgangsbohrung zur Aufnahme des Gewindeabschnitts der zugehörigen Pedikelschraube hat. Da der zylindrische Körper den Pedikelquerschnitt bis hin zur kortikalen Randschicht ausfüllt, hat er einen von der Kreisform abweichenden Querschnitt, sodass allein durch die zylindrische Formgebung eine Drehfixierung im Wirbel gegeben ist. Wenn die Pedikelschraube in den Spacer eingeschraubt wird, kann sich der Gewindeabschnitt der Pedikelschraube nach allen Seiten an der kortikalen Randschicht des Pedikels abstützen und dabei insbesondere die Biegefestigkeit der Verankerung in der cranio-caudalen Ebene wesentlich verbessern.

Zur Präparation kann der Pedikel dabei entweder aufgebohrt oder aufgefräst werden, wobei in diesem Fall vorzugsweise eine Bohrschablone oder sonstige Werkzeugschablone Anwendung findet. Alternativ ist auch eine Präparation mit Hilfe eines Ultraschallosteotoms möglich.

Das erfindungsgemäße Pedikelaugmentat, welches in einfacher oder mehrfacher Ausführung mit der Pedikelschraube zusammenwirkt, füllt in achsparalleler Ausrichtung zur Pedikelschraube den zwischen der Pedikelschraube und der Kortikalis verbleibenden Raum des Pedikels aus, sodass sich der Gewindeabschnitt auf der einen Seite unmittelbar an mehreren Punkten und auf der anderen Seite über das Pedikelaugmentat über mehrere Punkte unter damit rundum an der Kortikalis des Pedikels abstützt. Die eingangs erwähnte Anhebung des durch die Verankerung der Pedikelschraube bereitgestellten Biege-Stützmoments wird auf diese Weise optimal sichergestellt, ohne die Gefahr von Knochenfrakturen bzw. -beschädigungen anheben zu müssen.

Mit der Weiterbildung des Anspruchs 2 wird die Pedikelschraube nicht nur quer zum Pedikel, sondern auch in Längsrichtung noch besser im Pedikel verspannt.

Um die achsparallele Ausrichtung zwischen Pedikelschraube und Pedikelaugmentat nicht nur bei der Montage sondern auch im eingesetzten Zustand sicher zu fixieren, erstrecken sich das zumindest eine Pedikelaugmentat und die Pedikelschraube durch eine gemeinsame Verbindungslasche, in der entsprechende axiale Durchbrüche zur formschlüssigen Aufnahme eines Kopfabschnitts des Pedikelaugmentats und eines unterhalb des Schraubenkopfs liegenden Führungsabschnitts der Pedikelschraube ausgebildet sind. Der Verbindungslasche kann dabei in vorteilhafter Weise die weitere Funktion übertragen werden, der Pedikelschraube beim Eindrehen eine zusätzliche Führung zu geben, um auf diese Weise die Positionierungsgenauigkeit der Pedikelschraube zu verbessern. In vorteilhafter Weise weist das Pedikelaugmentat im Zusammenwirken mit der Verbindungslasche eine solche Konfiguration auf, dass im eingedrehten Zustand des Augmentats eine axiale Verriegelung zwischen Verbindungslasche und Pedikelaugmentat gegeben ist. Auf diese Weise kann die Verbindungslasche auch über das Pedikelaugmentat axial positioniert, beispielsweise in fester Anlage mit der knöchernen Struktur gehalten werden, wodurch gleichzeitig die Belastbarkeit des Implantats weiter gesteigert wird.

In einer besonders einfachen Ausgestaltung werden die axialen Durchbrüche von sich überschneidenden kreiszylindrischen Durchbrüchen, beispielsweise von kreiszylindrischen Bohrungen, gebildet.

Mit der Weiterbildung des Anspruchs 5 gelingt es, der Pedikelschraube über die Verbindungslasche eine definierte und räumlich fixierte Anschlagposition zur Verfügung zu stellen.

Eine besonders einfache axiale Sicherung zwischen Verbindungslasche und Pedikelaugmentat ist Gegenstand des Unteranspruchs 6. Durch diese Weiterbildung wird die Montage des Pedikelaugmentats zusammen mit der Verbindungslasche besonders einfach.

Eine weitere, montagetechnisch sehr einfache Möglichkeit der axialen Fixierung von Pedikelaugmentat und Verbindungslasche ist Gegenstand des Anspruchs 8.

Über die Weiterbildung des Anspruchs 9 lässt sich der Kraftfluss zwischen Pedikelaugmentat und Pedikelschraube zusätzlich optimieren. Durch die Gestaltung des Gewindeeingriffs zwischen dem Gewinde im Pedikelaugmentat und dem Gewindeabschnitt der Pedikelschraube kann einerseits Einfluss auf die radiale und/oder axiale Verspannung der beiden Komponenten genommen werden. Darüber hinaus ist es beispielsweise durch die Weiterbildung des Anspruchs 10 möglich, die achsparallele Ausrichtung von Pedikelschraube und Pedikelaugmentat über die gesamte Länge der achsparallelen Erstreckung zu stabilisieren, wodurch sich eine Verbesserung der Montagesituation und der Implantationssicherheit ergibt.

Gegenstand der Erfindung ist darüber hinaus ein Bausatz für ein System zur Verankerung einer Pedikelschraube in einem Wirbel eines Patienten, wobei dieser Bausatz aus einer Pedikelschraube, beispielsweise einer Standard-Pedikelschraube und einem Spacer nach einem der Ansprüche 1 bis 11 besteht. Ergänzend kann der Bausatz eine Verbindungslasche nach einem der Ansprüche 3 bis 8 aufweisen.

Schließlich ist gesonderter Gegenstand der vorliegenden Erfindung, für den auch gesondert Schutz beansprucht wird, ein Spacer gemäß einem der Ansprüche 14 bis 17.

Weitere vorteilhafte Ausgestaltungen sind Gegenstand der übrigen. Unteransprüche.

Nachstehend werden anhand schematischer Zeichnungen mehrere Ausführungsbeispiele der Erfindung näher erläutert. Es zeigen:
Fig. 1 ausschnittsweise eine Röntgenaufnahme einer Wirbelbogenwurzel (eines Pedikels);
Fig. 1A die Einzelheit IA einer computertomographischen Darstellung des Querschnitts in diesem Bereich;
Fig. 1B die computertomographische Darstellung der Einzelheit IB im Querschnitt;
Fig. 2 die Draufsicht auf einen Wirbel mit einseitig montierter Pedikelschraube;
Fig. 3 die Ansicht des Schnitts III-III in Fig. 2;
Fig. 4 die Ansicht des Schnitts gemäß IV-IV in Fig. 2;
Fig. 5 die Ansicht eines Wirbels mit präparierten Pedikeln für den Einsatz eines Spacers, der nicht Gegenstand der Erfindung ist;
Fig. 6 die Seitenansicht eines Pedikelaugmentats gemäß einer ersten Ausführungsform der Erfindung;
Fig. 7A eine maßstabsgetreue Darstellung eines Längsschnitts des Pedikelaugmentats gemäß Fig. 6;
Fig. 7B eine Seitenansicht des Pedikelaugmentats gemäß Fig. 7A;
Fig. 7C in vergrößertem Maßstab eine Stirnansicht des Pedikelaugmentats gemäß Fig. 6;
Fig. 8 eine Ansicht zur Verdeutlichung der lagemäßigen Zuordnung zwischen Pedikelschraube und Pedikelaugmentat gemäß Fig. 6 im montierten Zustand;
Fig. 9 eine perspektivische Darstellung des Pedikelaugmentats gemäß Fig. 6;
Fig. 10 eine perspektivische Ansicht einer Verbindungslasche zwischen einem Pedikelaugmentat gemäß Fig. 6 und einer in Verbindung damit zu benutzenden Pedikelschraube;
Fig. 11 eine perspektivische Ansicht der Zusammenstellung zwischen Pedikelaugmentat und Verbindungslasche im vormontierten Zustand;
Fig. 12 die Draufsicht der Zusammenstellung gemäß Fig. 11;
Fig. 13A und Fig. 13B in vergrößerter Darstellung die Zusammenstellung von Pedikelaugmentat und Verbindungslasche im fertigmontierten Zustand vor dem Eindrehen der Pedikelschraube;
Fig. 14A und 14B unterschiedliche Ansichten eines Wirbels mit beidseitig montierten Pedikelschrauben und erfindungsgemäßem Pedikelaugmentat;
Fig. 15A, 15B Ansichten eines Wirbels mit beidseitig montierten Pedikelschrauben und erfindungsgemäßem Pedikelaugmentat bei abgeschnittenem Wirbelbogen;
Fig. 16 einen Längsschnitt durch den Wirbel mit eingesetzter Pedikelschraube und erfindungsgemäßem Pedikelaugmentat;
Fig. 17 in vergrößerter Darstellung eine Variante des erfindungsgemäßen Pedikelaugmentats;
Fig. 18 eine Zusammenstellung einer modifizierten Ausführungsform des Pedikelaugmentats mit montierter Verbindungslasche gemäß einer weiteren Ausführungsform;
Fig. 19A und 19B unterschiedliche Darstellungen des Pedikelaugmentats gemäß Fig. 18 und eingesetzter Pedikelschraube;
Fig. 20 eine perspektivische Ansicht einer weiteren Ausführungsform eines erfindungsgemäßen Pedikelaugmentats;
Fig. 21 eine Seitenansicht einer zugehörigen Pedikelschraube; und
Fig. 22 eine schematische Ansicht der Zusammenstellung von Pedikelaugmentat gemäß Fig. 20 und Pedikelschraube gemäß Fig. 21.

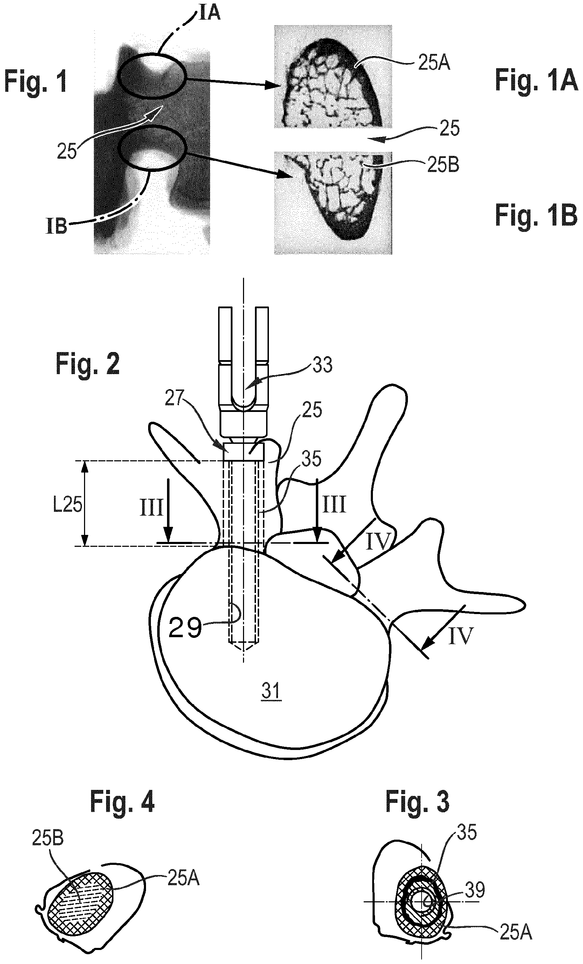

Das Röntgenbild gemäß Fig. 1 und die vergrößerten Computertomographiebilder der Einzelheiten IA und IB gemäß Fig. 1A und Fig. 1B lassen erkennen, dass der mit dem Bezugszeichen 25 bezeichnete Pedikel eines lumbalen und thorakalen Wirbels eine annäherungsweise ovale Querschnittsform hat. Die Fign. 1A und 1B lassen ferner erkennen, dass der Pedikel 25 eine äußere harte Knochenschicht 25A hat, die einen innen liegende schwammartige Knochensubstanz, das heißt die Spongiosa 25B umgibt. Dieser Zusammenhang ist auch aus der Darstellung gemäß Fig. 4 ersichtlich, die einen Schnitt des Pedikels senkrecht zu seiner Erstreckung darstellt.

Die Darstellungen nach den Fign. 1 und 4 lassen ferner erkennen, dass der ovale Querschnitt so gelegt ist, dass die längere Erstreckung in cranio-caudaler Richtung liegt.

In der Wirbelsäulenchirurgie wird häufig ein thorakolumbaler Wirbelsäulenfixateur benötigt, mit dem ausgewählte Wirbelgelenke dadurch passiviert werden, dass in Wirbel eingeschraubte Pedikelschrauben in der Ausgestaltung als Mono- oder Polyaxialschrauben unter Zuhilfenahme von Verbindungsstäben in ihrer Relativlage zueinander fixiert werden. Fig. 2 zeigt die Draufsicht eines Wirbels mit einseitig eingeschraubter Pedikelschraube 27, die sich mit ihrem Gewindeabschnitt 29 durch den Pedikel 25 bis hinein in der Wirbelkörper 31 erstreckt. Mit dem Bezugszeichen 33 ist die sogenannte "Tulpe" der Pedikelschraube 27 bezeichnet, in der der nicht näher dargestellte Verbindungsstab fixiert wird, wobei durch die Fixierung gleichzeitig das Gelenk zwischen Tulpe 33 und beispielsweise kugelförmigem Pedikelschraubenkopf arretiert wird.

Um den Gewindeabschnitt 29 der Pedikelschraube 27 rundum an der kortikalen Randschicht 25A, das heißt rundum abzustützen, ist der Raum zwischen dem Gewindeabschnitt 29 und der kortikalen Randschicht 25A des Pedikels 25 von einem Spacer 35 ausgefüllt, der in der gezeigten Ausführungsform von einem zylindrischen Körper gebildet ist, der sich über die gesamte Länge L 25 (siehe Fig. 2) erstreckt.

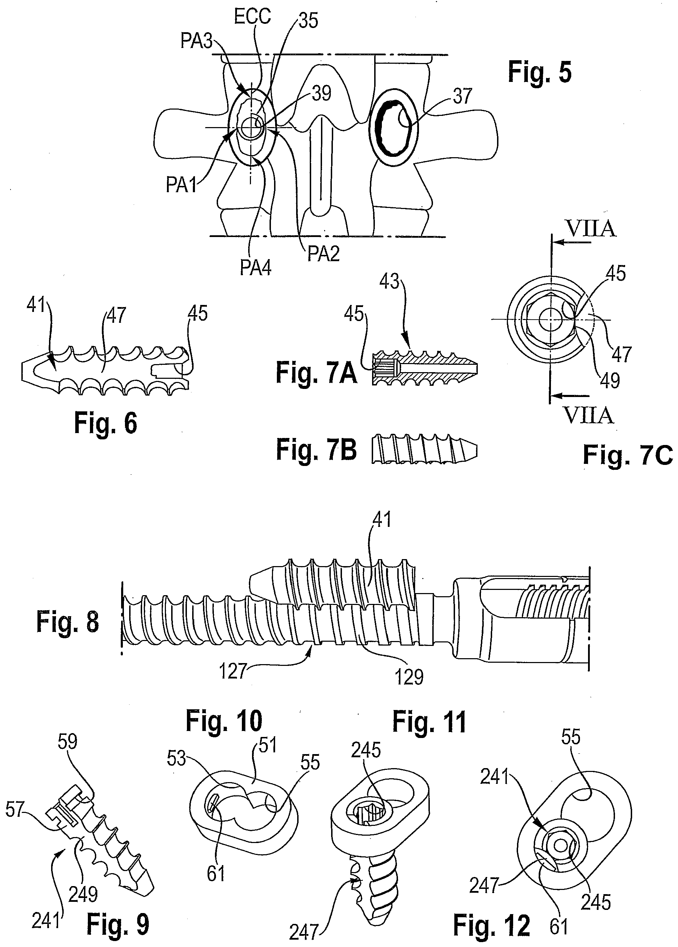

Aufgrund der aus Fig. 1 ersichtlichen Struktur des Pedikelquerschnitts ergibt sich, dass auch der zylindrische Körper einen von der Kreisform abweichenden Querschnitt, nämlich eine elliptischen Querschnitt hat, was aus den Darstellungen gemäß Fig. 3 und 5 hervorgeht. Im Einzelnen ist der zylindrische Körper in einer in Fig. 5 gezeigten präparierten, das heißt in den Pedikel 25 eingearbeiteten axialen Ausnehmung 37 formschlüssig aufgenommen. Die Ausnehmung 37 wird vorzugsweise unter Zuhilfenahme einer Bohrschablone oder eines anderen Werkzeugs in den Wirbel eingefräst. Alternativ ist auch eine Präparation mit Hilfe eines Ultraschallosteotoms möglich. Vorzugsweise ist die Außenoberfläche des Spacers 35 mit einer Riffelung bzw. einer Mikroriffelung ausgestattet, um die Verzahnung über dem gesamten Umfang mit der Innenoberfläche der Ausnehmung 37 zu verbessern.

Durch den Spacer 35 erstreckt sich eine axiale Durchgangsbohrung, im gezeigten Beispiel eine Gewindebohrung 39 zur Aufnahme des Gewindeabschnitts 29 der Pedikelschraube 27.

Der Spacer besteht beispielsweise aus Kunststoff, vorzugsweise aus einem PEEK-Kunststoff, weil dieser Werkstoff sich durch eine besondere biologische Verträglichkeit und Langlebigkeit bei gleichzeitig hoher Festigkeit auszeichnet. Um die Verbindung zwischen Spacer und Knochenstruktur dauerhaft zu verbessern, ist es von Vorteil, den Spacer geeignet zu beschichten, beispielsweise mit einer porösen Titan-Beschichtung auszustatten, wie sie von der Anmelderin Aesculap AG unter der Bezeichnung Plasmapore angeboten wird. Auch andere Materialien können eingesetzt werden, wie zum Beispiel Titan oder eine Titanlegierung, synthetische Knochenersatzstoffe oder allogenes Knochenmaterial. Ferner kann als Werkstoff Collagen, eine gepresste textile Struktur oder andere Gitterstrukturen eingesetzt werden, wie sie auf dem Gebiet der Implantattechnik allgemein verwendet werden und mittlerweile aus Kunststoff oder metallischem Werkstoff in jeder gewünschten Formgebung und mit beliebiger Gitterorientierung und -dimensionierung nach einem 3-D-Druckverfahren bzw. nach einem Laser-Sinterverfahren herstellbar sind.

Aus der Darstellung gemäß Fig. 5 und 3 wird klar, dass aufgrund der Verankerung der Pedikelschraube neben der bereits bei herkömmlichen Lösungen groben Stabilisierung des Gewindeabschnitts über die Abstützpunkte PA1 und PA2 eine zusätzliche Abstützung in cranio-caudaler Richtung, das heißt im Bereich der Ebene ECC, das heißt im Bereich der Punkte PA3, PA4 gegeben ist, wodurch sich somit eine Art Rundum-Abstützung der Pedikelschraube an der kortikalen Randschicht 25A des Pedikels 25 ergibt. Querkräfte, insbesondere solche, die in cranio-caudaler Richtung wirken, und Biegemomente können auf diese Weise wesentlich besser als herkömmlich aufgenommen werden, sodass die Verbindung zuverlässig gegen Auslockern der Pedikelschraube gesichert ist.

Nachstehend wird eine erste Ausführungsform der Erfindung unter Bezugnahme auf die Fign. 6 bis 16 näher erläutert.

Bei der erfindungsgemäßen Ausführungsform übernimmt die Aufgabe des vorher unter Bezug auf die Figuren 1 bis 5 beschriebenen Spacers 35 ein im Wesentlichen zylindrisches sogenanntes Pedikelaugmentat 41, welches achsparallel zur Pedikelschraube in den Pedikel eingesetzt wird. Das im Wesentlichen zylindrische Pedikelaugmentat 41 hat im Wesentlichen die Formgebung einer Knochenschraube oder eines Knochendübels, das heißt es ist auf der Außenseite mit einem Verankerungsgewinde 43 ausgestattet und es hat auf der dem Schraubenkopf der Pedikelschraube 127, das heißt an der proximalen Stirnseite eine Mehrkantausnehmung, beispielsweise eine Sechskantausnehmung 45 für das entsprechend Einschraubwerkzeug.

Wie aus der Darstellung gemäß Fig. 8 ersichtlich ist, hat das im Wesentlichen zylindrische Pedikelaugmentat 41 auf der im Gewindeabschnitt 129 zugewandten Seite eine zylindrische Ausnehmung 47, die sich -wie aus Fig. 6 ersichtlich - im Wesentlichen über die gesamte Länge des Pedikelaugmentats 41 erstreckt. Man erkennt aus der Darstellung gemäß Fig. 7C, dass der Querschnitt der zylindrischen Ausnehmung 47 im Wesentlichen linsenartig ist.

Die Formgebung der zylindrischen Ausnehmung 47 ist so gewählt, dass sie mit der Einhüllenden des Gewindeabschnitts 129 der Pedikelschraube 127 zusammenfällt. Mit anderen Worten, der Gewindeabschnitt 129 schmiegt sich im eingesetzten Zustand von Pedikelaugmentat 41 und Pedikelschraube 127 an die Innenwandung der zylindrischen Ausnehmung 47 an. Auf diese Weise kann sich der Gewindeabschnitt 129 beim Einschrauben an Pedikelaugmentat 41 mit radialer Verspannung abstützen.

Pedikelaugmentat 41 und Pedikelschraube 127 werden so gesetzt, dass die Spongiosa im Pedikel 25 von diesen beiden Komponenten so vollständig wie möglich ausgefüllt wird. Mit anderen Worten, die Pedikelschraube wird mehr kranial oder caudal positioniert als bei der Ausführungsform gemäß Fig. 1 bis 5 gezeigt, sodass sich die Pedikelschraube im cranialen oder caudalen Bereich des Pedikels über mehrere Punkte am Umfang an der kortikalen Schicht des Pedikelquerschnitts abstützen kann. Über die zylindrische Ausnehmung 47 mit der Stützfläche 49 kann der Gewindeabschnitt 129 aber auch unter Zwischenschaltung des Pedikelaugmentats 41 eine Abstützung auf der anderen Seite der Pedikelschraube erhalten, sodass erneut eine Art Rundum-Abstützung des Gewindeabschnitts 129 an der Kortikalis des Pedikels 25 bereitgestellt ist.

Über die Stützfläche 49 der zylindrischen Ausnehmung 47 erfolgt darüber hinaus eine Fixierung der beiden Komponenten in achsparalleler Ausrichtung. Zur Implantation verwendet man Hilfsvorrichtungen, um den exakten Achsabstand zwischen Pedikelschraube 127 und Pedikelaugmentat 41 einzustellen. Die Planung der Positionierung kann dabei über ein konventionelles Röntgen beispielsweise intraoperativ erfolgen. Besonders bevorzugt werden die Komponenten mithilfe eines Navigationssystems eingebracht.

Eine beispielhafte Einbringung erfolgt dadurch, dass zunächst das Augmentat 41 gesetzt wird, wobei die erforderliche Bohrung angekörnt, dann ein Vorbohren und ein eventuelles Gewindeschneiden vorgenommen wird. Sobald das Augmentat 41 gesetzt ist, kann die Bohrvorrichtung für die Bohrung der Pedikelschraube 129 in Funktion treten. Die Bohrvorrichtung stütz sich vorteilhafterweise dann über einen sogenannten K-Draht am Augmentat 41 ab und gibt so die Bohrung für die Pedikelschraube 129 vor. Das Eindrehen der Pedikelschraube 127 erfolgt dann erneut über einen gesetzten K-Draht.

Man erkennt am besten aus der Darstellung gemäß Fig. 8, dass beim Eindrehen der Pedikelschraube 127 der Gewindeabschnitt 129 in ständigem Anlagekontakt mit der Stützfläche 49 des Pedikelaugmentats 41 steht. Durch geeignete Gestaltung der Stützfläche 49 in Anpassung an den Gewindeabschnitt 129 einhüllende Fläche kann die radiale Verspannung zwischen Gewindeabschnitt 129 und Pedikelaugmentat 41 in gewünschter Weise gesteuert werden.

Bei der gezeigten Ausgestaltung gemäß Fig. 6 bis 8 ist die Stützfläche 49 glatt ausgebildet, sodass mit den Gewindegängen des Gewindeabschnitts 129 keine Verzahnung stattfindet. In Abwandlung der gezeigten Ausführungsform kann jedoch in diesem Bereich zusätzlich eine Profilierung eingebracht werden, mit der es gelingt, im Bereich der Stützfläche 49 auch für eine zusätzliche axiale Verspannung zwischen Gewindeabschnitt 129 und Pedikelaugmentat 41 zu sorgen.

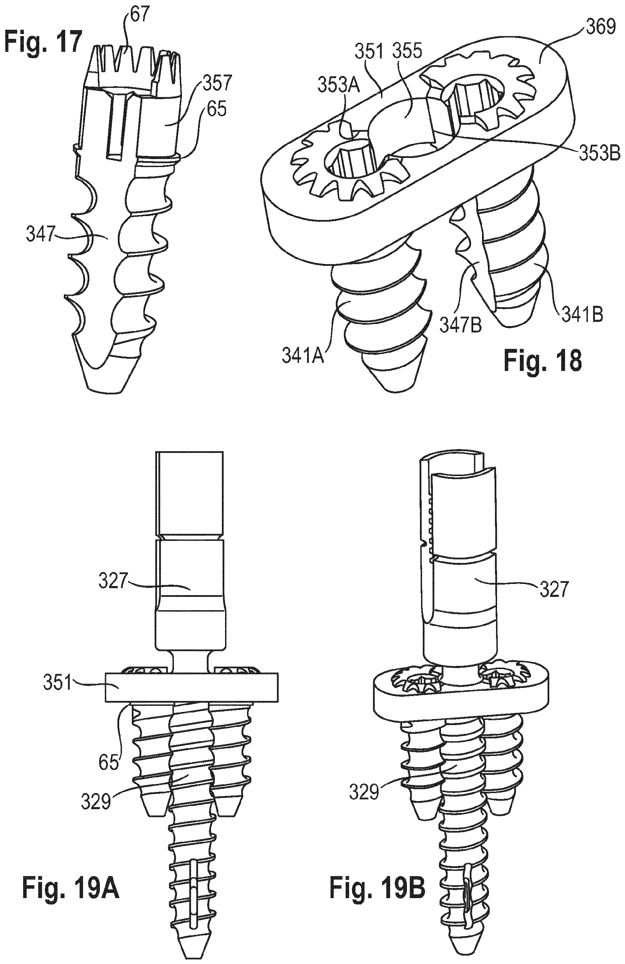

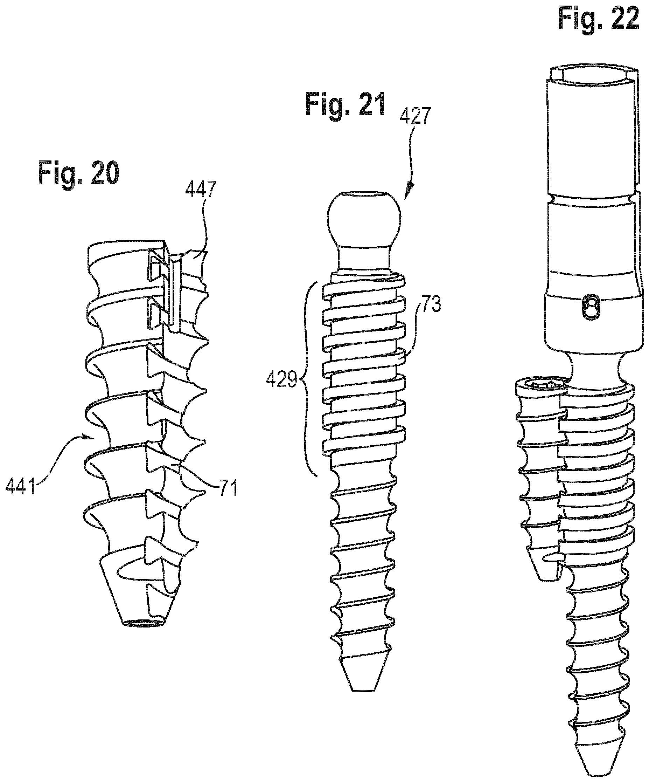

Im Folgenden werden Ausführungsbeispiele des erfindungsgemäßen Systems zur Verankerung einer Pedikelschraube in einem Wirbel beschrieben, mit denen eine feste Verbindung zwischen der Pedikelschraube einerseits und dem Augmentat andererseits sichergestellt wird. Bei der Beschreibung dieser Varianten werden für diejenigen Komponenten, die den Elementen der zuvor beschriebenen Ausführungsbeispiele entsprechen, ähnliche Bezugszeichen verwendet, denen jedoch eine "2" (Fig. 9 bis 16), "3" (Fig. 17 bis 19) bzw. "4" (Fig. 20 bis 22) vorangestellt ist.

Bei der Ausführungsform gemäß Fig. 9 bis 16 werden Pedikelschraube und Pedikelaugmentat über eine Verbindungslasche 51 verbunden, durch die sich einerseits das Pedikelaugmentat 241 und die Pedikelschraube 227 erstrecken. Das Pedikelaugmentat 241 ist - mit Ausnahme des Augmentatkopfs - in der gleichen Weise gestaltet wie das Pedikelaugmentat 41 der Ausführungsform gemäß Fig. 6 bis 8, das heißt es hat erneut einen im Wesentlichen halbmondförmigen Querschnitt, der durch die zylindrische Ausnehmung 247 entsteht. Der vom Querschnitt des Pedikelaugmentats 241 herausgeschnittene Bereich 247 ist am besten der Fig. 12 entnehmbar.

Für die formschlüssige Aufnahme von Pedikelaugmentat 241 und Pedikelschraube 227 sind in der Verbindungslasche 51 zwei achsparallele axiale Durchbrüche 53 und 55 ausgebildet, wobei in der gezeigten Ausführungsform diese axialen Durchbrüche von sich überschneidenden kreiszylindrischen Durchbrüchen gebildet sind. Der Durchbruch 53 dient zur Aufnahme eines kreiszylindrischen Kopfabschnitts 57 des Pedikelaugmentats 241, das - wie bei der Ausführungsform gemäß Fig. 6 bis 8 - mit einer Innen-Mehrkantausnehmung 245 ausgestattet ist. Auf der Außenseite trägt der Kopfabschnitt 57 eine mit 59 bezeichnete Ringsegmentnut, in die formschlüssig ein Sicherungsvorsprung 61 eingreifen kann, der von der Innenoberfläche des Durchbruchs 53 der Verbindungslasche 51 vorsteht.

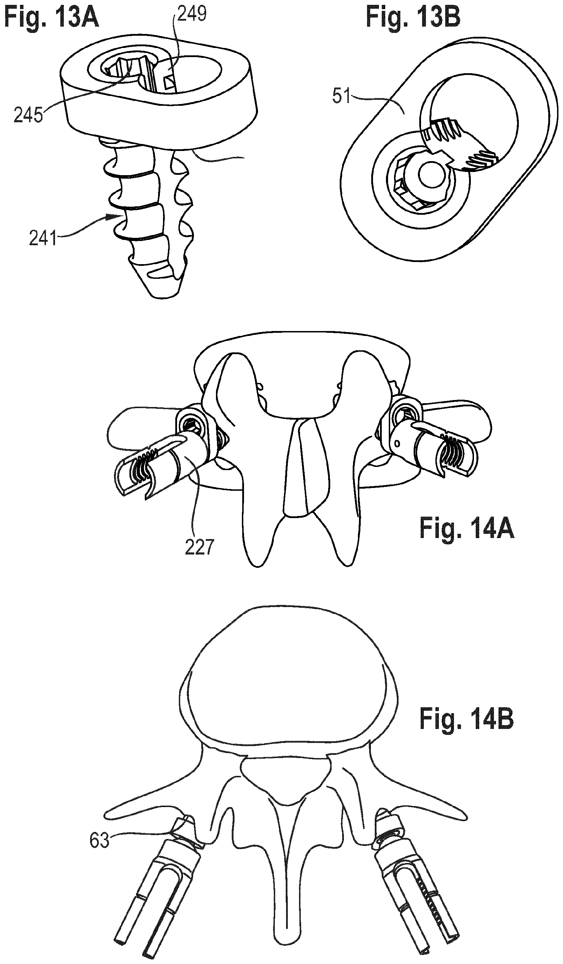

Der Sicherungsvorsprung 61 hat - wie am besten aus der Fig. 12 ersichtlich ist - eine von der Innenoberfläche vorstehende Fläche, die kleiner ist als der linsenförmige Querschnitt (siehe Fig. 12) der Ausnehmung 247 im Pedikelaugmentat 241. Aufgrund dieser Gestaltung ist eine freie axiale Bewegung zwischen Pedikelaugmentat 241 und Verbindungslasche 51 gegeben, solange der Sicherungsvorsprung 41 nicht in die Ringsegmentnut 59 des Kopfabschnitts 57 des Pedikelaugmentats 41 eintaucht. Bei der Montage geht man deshalb so vor, dass das Augmentat 241 in eine entsprechend präparierte Bohrung bzw. Gewindebohrung so weit eingeschraubt wird, dass beim Aufsetzen der Verbindungslasche 51 in der in Fig. 12 gezeigten Position bei einem Weiterdrehen des Pedikelaugmentats 241 und damit einhergehender axialer Versetzung des Pedikelaugmentats der Sicherungsvorsprung 61 in die Ringsegmentnut 59 eintauchen kann und noch um etwa 180° verdreht werden kann, bis das Pedikelaugmentat 241 wie in Fig. 13A und Fig. 13B gezeigte Position erreicht, in der die Stützfläche 249 das Kreissegment des kreiszylindrischen Durchbruchs 55 zu einem Vollkreis ergänzt und die bodenseitige Fläche 63 der Verbindungslasche 51 in fester Anlage mit dem Wirbelknochen steht. In dieser Position, die in den Fign. 13A und 13B gezeigt ist, kann mit dem Positionieren bzw. Einschrauben der Pedikelschraube 227 begonnen werden. Über den kreiszylindrischen Durchbruch 55 kann die Pedikelschraube 227 geführt und in den letzten Montageschritten im exakten Achsabstand zum Pedikelaugmentat 241 fixiert werden, was in den Fign. 14 bis 16 dargestellt ist.

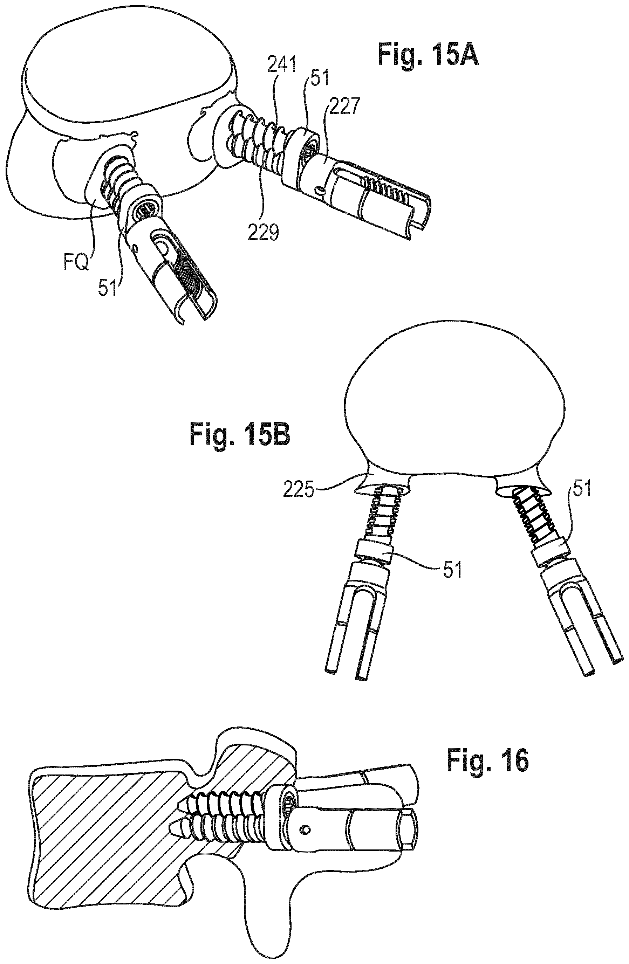

Man erkennt aus den Darstellungen gemäß Fig. 14A und 14B, dass das Pedikelaugmentat 241 in der Position mit der Verbindungslasche 51 verriegelt ist, in der sich letztere in fester Anlage mit einer Stirnfläche 63 des Wirbelbogens befindet. Es soll jedoch an dieser Stelle hervorgehoben werden, dass ein fester Kontakt mit einer solchen Stirnfläche nicht unbedingt erforderlich ist. Entscheidend ist viel mehr, dass - wie aus den Schnittansichten der Fign. 15A und 15B sowie aus der Darstellung gemäß Fig. 16 erkennbar - der Gewindeabschnitt 229 der Pedikelschraube 227 und der daran sich anschließende Querschnitt des Pedikelaugmentats 241 den Querschnitt FQ des Pedikels 225 so weit ausfüllen, dass mehr oder weniger die gesamte Spongiosa von diesen Querschnitten ausgefüllt wird und sich die Pedikelschraube 227 bzw. der Gewindeabschnitt 229 der Pedikelschraube 227 rundum an der Kortikalis des Pedikels 225 abstützen kann.

Die vorstehend beschriebenen Ausführungsformen der Fign. 6 bis 16 sind so aufgebaut, dass die Pedikelschraube mit einem einzigen Pedikelaugmentat 41 bzw. 241 zusammenwirkt, um den erfindungsgemäßen Effekt der Verankerung der Pedikelschraube im Pedikel sicherzustellen. Es ist jedoch gleichermaßen möglich, die Pedikelschraubenaugmentation unter Zuhilfenahme mehrerer Augmentate vorzunehmen.

In den Fign. 17 bis 19 ist eine Ausführungsform beschrieben, bei der zwei Pedikelaugmentate 341A und 341B cranial bzw. caudal bezüglich der Pedikelschraube 327 gesetzt werden. Zur Fixierung des Achsabstands zwischen Pedikelaugmentat 341A, B und Gewindeabschnitt 329 der Pedikelschraube 327 ist eine Verbindungslasche 351 vorgesehen, in der drei kreiszylindrische koaxiale Durchbrüche 353A, B für den Kopfabschnitt 357 der Pedikelaugmentate 341A, B und ein zentraler kreiszylindrischer Durchbruch 355 für die Pedikelschraube 327 ausgebildet sind.

Die axiale Fixierung zwischen Pedikelaugmentat 341A, B und Verbindungslasche 351 erfolgt bei der Ausführungsform nach den Fign. 17 bis 19 dadurch, dass der Kopfabschnitt 357 unter Auflage an einer Ringschulter des Kopfabschnitts 357 mit der Verbindungslasche 351 verstemmt wird. Zu diesem Zweck trägt der Kopfabschnitt 357 einen Ring von zungenartigen Vorsprüngen 67, die zum Verstemmen - wie in Fig. 18 gezeigt - umgebogen und in die obere Stirnfläche 369 hineingedrückt werden können.

Auch bei der Ausführungsform gemäß Fig. 17 bis 19 kann das Einsetzen der Pedikelschrauben derart erfolgen, dass die Verbindungslasche 351 an der Knochensubstanz anliegt. In diesem Fall ist es von Vorteil, zumindest den Bereich der Verbindungslasche 351, der mit der Knochensubstanz in Berührung kommt, mit einer entsprechenden Beschichtung zu versehen, um ein knöchernes Anwachsen zu ermöglichen.

Als Alternative zu der vorstehend beschriebenen Lagefixierung zwischen Pedikelaugmentat und Pedikelschraube in Form der Verbindungslasche 51 bzw. 351 oder zusätzlich zu dieser Lagefixierung wird nachfolgend unter Bezug auf die Fign. 20 bis 22 eine Alternative beschrieben. Diese besteht darin, dass das mit dem Bezugszeichen 441 bezeichnete Pedikelaugmentat im Bereich seiner zylindrischen Ausnehmung 447 mit einem hinterschnittenen Gewinde 71 ausgestattet wird, welches komplementär zu einem modifizierten Schraubengewinde 73 im Gewindeabschnitt 429 der Pedikelschraube 427 gestaltet ist. Die Maßstäbe der Darstellungen der Fign. 20 bis 22 sind unterschiedlich. Es versteht sich von selbst, dass der Gewindeabschnitt 429 eine axiale Länge hat, die mit der axialen des hinterschnittenen Gewindes 71 im Bereich des Pedikelaugmentats 441 übereinstimmt.

Mit dieser Gestaltung ist es möglich, bei gesetztem Pedikelaugmentat 441 die Pedikelschraube 427 (in der Ausgestaltung nach den Fign. 21 und 22 als Polyaxial-Pedikelschraube) in die präparierte und exakt gesetzte Vorbohrung des Pedikels einzuschrauben, sodass nach einer bestimmten Einschraubstrecke der Gewindegang des Schraubengewindes 73 in die hinterschnittenen Gewindesegmente des hinterschnittenen Gewindes 71 eingreift. Durch die Hinterschneidung des Gewindes werden die beiden Komponenten aneinander gekoppelt. Es kann sogar die Anordnung so getroffen sein, dass die beiden komplementären Gewindeabschnitte aufeinander zu vorgespannt werden. Diese Anordnung erlaubt es, auf eine Verbindungslasche 51 bzw. 351 zu verzichten.

Die vorstehend beschriebene erfindungsgemäße Pedikelaugmentation ist so ausgebildet, dass sie ohne eine Zementierung mit sogenanntem Knochenzement, das heißt polymerem Methyl-Metacrylat (PMMA) auskommt. Es ist jedoch möglich, die Pedikelaugmentation unter Zuhilfenahme von Zementaugmentationsschrauben vorzunehmen. Eine derartige Pedikelschraube ist in Fig. 19A und Fig. 19B mit 329 dargestellt. Mit einer solchen Schraube erfolgt eine herkömmliche Zementierung nur im Wirbelkörper, während im Pedikelkanal ausschließlich die vorstehend beschriebene Pedikelaugmentation zur Anwendung kommt. Dadurch ergibt sich der besondere Vorteil, dass keine Gefahr eines unkontrollierten Zementaustritts gegeben ist.

Mit der vorstehend beschriebenen Pedikelaugmentation ist auch wirksam ausgeschlossen, dass es zu einer lokalen Sprengung der Pedikelstruktur kommt. Die Gefahr einer Überbeanspruchung der Pedikelstruktur kann zusätzlich dadurch verringert werden, dass eine entsprechende Präparation des Wirbels vorgenommen wird.

Selbstverständlich sind Abweichungen von den gezeigten Ausführungsformen möglich, ohne den Grundgedanken der Erfindung zu verlassen. Alternativ zu den Ausführungsformen der Fign. 9 bis 19 können auch mehr Augmentate verwendet werden, die beispielsweise sternförmig gesetzt sind. Es ist auch möglich, bei Verwendung von zwei oder mehr Pedikelaugmentaten unterschiedliche Querschnitte zu verwenden, wenn auf diese Weise eine noch bessere Ausfüllung der Spongiosa des Pedikels erzielt wird. Selbstverständlich können für die einzelnen Komponenten der Pedikelaugmentation alle bislang im Bereich der Orthopädietechnik eingesetzten Werkstoffe angewendet werden.

Alternativ zu der Aufnahme des Kopfabschnitts 57 bzw .357 des Pedikelaugmentats 241, 341 kann auch ein konischer Kopfabschnitt oder ein gestufter Kopfabschnitt verwendet werden. Es ist auch möglich, das Pedikelaugmentat axial zu verkürzen, sodass es sich nur um einen vorbestimmten verkürzten Längsabschnitt des Pedikelkanals erstreckt.

Die Erfindung schafft somit ein System zur Verankerung einer Pedikelschraube in einem Wirbel eines Patienten, insbesondere im lumbalen oder thorakalen Bereich der Wirbelsäule. Das System hat eine Pedikelschraube, die einen im Wirbelbogen einschließlich des Pedikels versenkbaren Gewindeabschnitt aufweist. Der Raum zwischen Gewindeabschnitt und kortikaler Randschicht des Pedikels wird zumindest bereichsweise durch einen Spacer ausgefüllt, der so gestaltet bzw. bezüglich des Gewindeabschnitts im Pedikelquerschnitt positioniert ist, dass über dem zumindest einen Spacer eine Rundum-Abstützung des Gewindeabschnitts an der kortikalen Randschicht ermöglicht ist. Die Erfindung schafft ferner einen entsprechenden Bausatz für das System, wobei der Bausatz aus einer Standard-Pedikelschraube und einem Spacer besteht, der den Raum zwischen dem Gewindeabschnitt und kortikaler Randschicht des Pedikels zumindest bereichsweise ausfüllt. Schließlich schafft die Erfindung einen entsprechenden Spacer in Form eines zylindrischen Körpers mit im Wesentlichen ovalem Querschnitt zur formschlüssigen Einpassung in einer entsprechenden in den Pedikel eingearbeiteten axialen Ausnehmung und mit einer axialen Durchgangsbohrung zur Aufnahme des Gewindeabschnitts einer Pedikelschraube, sowie einen Spacer in Form eines im Wesentlichen zylindrischen Pedikelaugmentats, das die Formgebung einer Knochenschraube oder eines Knochenbügels und auf einer Seite eine zylindrische Ausnehmung zur radialen Abstützung eines Gewindeabschnitts einer Pedikelschraube hat.

## Patentansprüche

1. System zur Verankerung einer Pedikelschraube (127; 327; 427) in einem Wirbel eines Patienten, insbesondere im lumbalen oder thorakalen Bereich der Wirbelsäule, mit einer Pedikelschraube (127; 327; 427), die einen im Wirbelbogen einschließlich des Pedikels (25) versenkbaren Gewindeabschnitt (129; 229; 329; 429) aufweist, und zumindest einem den Raum zwischen Gewindeabschnitt (129; 229; 329; 429) und kortikaler Randschicht (25A) des Pedikels zumindest bereichsweise ausfüllenden Spacer (41; 241; 341A, B; 441), **dadurch gekennzeichnet, dass** der zumindest eine Spacer (41; 241; 341A, B; 441) von einem achsparallel versetzt zur Pedikelschraube (127; 327; 427) in den Wirbel einsetzbaren im Wesentlichen zylindrischen Pedikelaugmentat (41; 241; 341A, B; 441) gebildet ist, das im Wesentlichen die Formgebung einer Knochenschraube oder eines Knochendübels und auf der der Pedikelschraube (27; 127; 227; 327; 427) zugewandten Seite der Außenumfangsoberfläche eine zylindrische Ausnehmung (47; 247; 347) hat, an der sich der Gewindeabschnitt (129; 229; 329; 429) mit radialer Verspannung abstützt, wodurch eine Rundum-Abstützung des Gewindeabschnitts (129; 229; 329; 429) an der kortikalen Randschicht (25A) ermöglicht ist.

2. System nach Anspruch 1, **dadurch gekennzeichnet, dass** sich der Gewindeabschnitt (129; 229; 329; 429) am Pedikelaugmentat (41; 241; 341A, B; 441) zur Ermöglichung einer zusätzlichen axialen Verspannung über eine Stützfläche (49) der zylindrischen Ausnehmung (47; 247; 347) mit eingebrachter Profilierung abstützt.

3. System nach Anspruch 1 oder 2, **dadurch gekennzeichnet, dass** sich das zumindest eine Pedikelaugmentat (241; 341A, B; 441) und die Pedikelschraube (227; 327) durch eine gemeinsame Verbindungslasche (51; 351) erstrecken, in der axiale Durchbrüche (53, 55; 3353A,B, 355) zur formschlüssigen Aufnahme eines Kopfabschnitts (57; 357) des Pedikelaugmentats (241; 341A, B; 441) und eines unterhalb des Schraubenkopfs liegenden Führungsabschnitts (229; 329; 429) der Pedikelschraube (227; 327) ausgebildet sind.

4. System nach Anspruch 3, **dadurch gekennzeichnet, dass** die axialen Durchbrüche von sich überschneidenden kreiszylindrischen Durchbrüchen (53, 55; 3353A,B, 355) gebildet sind.

5. System nach Anspruch 3 oder 4, **dadurch gekennzeichnet, dass** die Verbindungslasche (51; 351) zur axialen Fixierung des Pedikelaugmentats (241; 341A, B) und gegebenenfalls als axialer Anschlag für die Pedikelschraube (227; 327) dient.

6. System nach Anspruch 5, **dadurch gekennzeichnet, dass** der axiale Durchbruch (53; 353A, B) für das Pedikelaugmentat (241; 341A, B) auf der dem Durchbruch (55; 355) für die Pedikelschraube (227; 327) abgewandten Seite einen Sicherungsvorsprung (61) trägt, der formschlüssig in einer Ringsegmentnut (59) des Kopfabschnitts (57) des Pedikelaugmentats (241; 341A, B) aufnehmbar ist.

7. System nach Anspruch 6, **dadurch gekennzeichnet, dass** der Sicherungsvorsprung (61) eine von der Innenwandung (53) radial vorstehende Fläche hat, die kleiner ist als der Querschnitt der zylindrischen Ausnehmung (47) im Pedikelaugmentat (241).

8. System nach Anspruche 5, **dadurch gekennzeichnet, dass** das zumindest eine Pedikelaugmentat (341A, 341B) mit der Verbindungslasche (351) verstemmbar ist.

9. System nach einem der Ansprüche 1 bis 8, **dadurch gekennzeichnet, dass** die zylindrische Ausnehmung (447) im Pedikelaugmentat (441) mit einem Gewinde (71) ausgestattet ist, welches mit dem Gewindeabschnitt (429) der Pedikelschraube (427) in Funktionseingriff bringbar ist.

10. System nach Anspruch 9. **dadurch gekennzeichnet, dass** das Gewinde (71) im Pedikelaugmentat (441) hinterschnitten ist.

11. System nach einem der Ansprüche 1 bis 10, **dadurch gekennzeichnet, dass** das Pedikelaugmentat (41; 241; 341A, B; 441) und/oder die Verbindungslasche (51; 351) aus einer Titanlegierung besteht und vorzugsweise zumindest bereichsweise eine poröse Titan-Beschichtung aufweist.

12. Bausatz für ein System zur Verankerung einer Pedikelschraube in einem Wirbel eines Patienten, mit einer Pedikelschraube (27; 127; 227; 327; 427) und einem Spacer (35; 41; 241; 341A, B; 441) nach einem der Ansprüche 1 bis 11.

13. Bausatz nach Anspruch 12, mit einer Verbindungslasche (51; 351) nach einem der Ansprüche 3 bis 8.

14. Spacer für ein System zur Verankerung einer Pedikelschraube in einem Wirbel eines Patienten, in Form eines im Wesentlichen zylindrischen Pedikelaugmentats (41; 241; 341A, B; 441), das im Wesentlichen die Formgebung einer Knochenschraube oder eines Knochendübels und auf einer Seite der Außenumfangsoberfläche eine zylindrische Ausnehmung (47; 247; 347; 447) zur radialen Abstützung eines Gewindeabschnitts (129; 229; 329; 429) einer Pedikelschraube (127; 227; 327; 427) hat.

15. Spacer nach Anspruch 14, mit einem kreiszylindrischen Kopfabschnitt (57), in dem eine Ringsegmentnut (59) zur Aufnahme eines axial wirkenden Verriegelungsteils (61) ausgebildet ist.

16. Spacer nach Anspruch 14, mit einem Kopfabschnitt (357), an dem angeformte Zungen (67) zur Verstemmung mit einem Montageteil (351) ausgebildet ist.

17. Spacer nach einem der Ansprüche 14 bis 16, **dadurch gekennzeichnet, dass** die zylindrische Ausnehmung (447) mit einem vorzugsweise hinterschnittenen Gewinde (71) für das Zusammenwirken mit einem Gewindeabschnitt (73) einer Pedikelschraube (427) ausgestattet ist.

## Claims

1. A system for anchoring a pedicle screw (127; 327; 427) in a vertebra of a patient, in particular in the lumbar or thoracic region of the spinal column, having a pedicle screw (127; 327; 427) which has a threaded portion (129; 229; 329; 429) which can be recessed in the vertebral arch including the pedicle (25), and having at least one spacer (41; 241; 341A, B; 441) which at least partially fills the space between the threaded portion (129; 229; 329; 429) and cortical edge layer (25A) of the pedicle, **characterised in that** the at least one spacer (41; 241; 341A, B; 441) is formed by means of a substantially cylindrical pedicle graft (41; 241; 341A, B; 441) which can be inserted into the vertebra so as to be offset in an axially parallel manner with respect to the pedicle screw (127; 327; 427) and which substantially has the shaping of a bone screw or a bone dowel and, at the side of the outer peripheral surface facing the pedicle screw (27; 127; 227; 327; 427) has a cylindrical recess (47; 247; 347) on which the threaded portion (129; 229; 329; 429) is supported with radial pretensioning, whereby an all-round support of the threaded portion (129; 229; 329; 429) on the cortical edge layer (25A) is enabled.

2. The system according to claim 1, **characterised in that** the threaded portion (129; 229; 329; 429) is supported on the pedicle graft (41; 241; 341A, B; 441) in order to enable an additional axial pretensioning by means of a support face (49) of the cylindrical recess (47; 247; 347) with the profiling introduced.

3. The system according to claim 1 or 2, **characterised in that** the at least one pedicle graft (241; 341A, B; 441) and the pedicle screw (227; 327) extend through a common connection flap (51; 351) in which axial apertures (53, 55; 353A, B, 355) are constructed to receive a head portion (57; 357) of the pedicle graft (241; 341A, B; 441) and a guiding portion (229; 329; 429) of the pedicle screw (227; 327) which is located below the screw head in a positive-locking manner.

4. The system according to claim 3, **characterised in that** the axial apertures are formed by means of intersecting circular-cylindrical apertures (53, 55; 353A, B, 355).

5. The system according to claim 3 or 4, **characterised in that** the connection flap (51; 351) serves to axially fix the pedicle graft (241; 341A, B) and where applicable acts as an axial stop for the pedicle screw (227; 327).

6. The system according to claim 5, **characterised in that** the axial aperture (53; 353A, B) for the pedicle graft (241; 341A, B) carries, at the side facing away from the aperture (55; 355) for the pedicle screw (227; 327), a securing projection (61) which can be received in a positive-locking manner in an annular segment groove (59) of the head portion (57) of the pedicle graft (241; 341A, B).

7. The system according to claim 6, **characterised in that** the securing projection (61) has a face which protrudes radially from the inner wall (53) and which is smaller than the cross-section of the cylindrical recess (47) in the pedicle graft (241).

8. The system according to claim 5, **characterised in that** the at least one pedicle graft (341A, 341B) can be caulked with the connection flap (351).

9. The system according to any one of claims 1 to 8, **characterised in that** the cylindrical recess (447) in the pedicle graft (441) is provided with a thread (71) which can be brought into functional engagement with the threaded portion (429) of the pedicle screw (427).

10. The system according to claim 9, **characterised in that** the thread (71) is undercut in the pedicle graft (441).

11. The system according to any one of claims 1 to 10, **characterised in that** the pedicle graft (41; 241; 341A, B; 441) and/or the connection flap (51; 351) comprises a titanium alloy and preferably at least partially has a porous titanium coating.

12. A construction kit for a system for anchoring a pedicle screw in a vertebra of a patient, having a pedicle screw (27; 127; 227; 327; 427) and a spacer (35; 41; 241; 341A, B; 441) according to any one of claims 1 to 11.

13. The construction kit according to claim 12, having a connection flap (51; 351) according to any one of claims 3 to 8.

14. A spacer for a system for anchoring a pedicle screw in a vertebra of a patient, in the form of a substantially cylindrical pedicle graft (41; 241; 341A, B; 441) which substantially has the shaping of a bone screw or a bone dowel and, at one side of the outer peripheral surface, has a cylindrical recess (47; 247; 347; 447) for radially supporting a threaded portion (129; 229; 329; 429) of a pedicle screw (127; 227; 327; 427).

15. The spacer according to claim 14, having a circular-cylindrical head portion (57) in which an annular segment groove (59) for receiving an axially acting locking component (61) is formed.

16. The spacer according to claim 14, having a head portion (357) on which formed-on tongues (67) are formed for caulking with an assembly component (351).

17. The spacer according to any one of claims 14 to 16, **characterised in that** the cylindrical recess (447) is provided with a preferably undercut thread (71) for cooperation with a threaded portion (73) of a pedicle screw (427).

## Revendications

1. Système d'ancrage d'une vis pédiculaire (127 ; 327 ; 427) dans une vertèbre d'un patient, en particulier dans la zone lombaire ou thoracique de la colonne vertébrale, avec une vis pédiculaire (127 ; 327 ; 427), qui présente une section filetée (129 ; 229 ; 329 ; 429) escamotable dans l'arc vertébral, pédicule (25) compris, et au moins une entretoise (41 ; 241 ; 341A, B ; 441) remplissant au moins par zone l'espace entre la section filetée (129 ; 229 ; 329 ; 429) et la couche superficielle corticale (25A) du pédicule, **caractérisé en ce que** l'au moins une entretoise (41 ; 241 ; 341A, B ; 441) est formée par une augmentation pédiculaire (41; 241; 341A, B ; 441) sensiblement cylindrique insérable dans la vertèbre en parallélisme axial par rapport à la vis pédiculaire (127 ; 327 ; 427), qui a sensiblement la forme d'une vis d'ostéosynthèse ou d'une cheville osseuse, et un évidement cylindrique (47 ; 247 ; 347) sur le côté de la surface circonférentielle extérieure tournée vers la vis pédiculaire (27 ; 127 ; 227 ; 327 ; 427), , sur lequel la section filetée (129 ; 229 ; 329 ; 429) s'appuie avec serrage radial, ce qui permet un appui entier de la section filetée (129 ; 229 ; 329 ; 429) sur la couche superficielle corticale (25A).

2. Système selon la revendication 1, **caractérisé en ce que** la section filetée (129 ; 229 ; 329 ; 429) s'appuie sur l'augmentation pédiculaire (41 ; 241 ; 341A, B ; 441) pour permettre un serrage axial supplémentaire par le biais d'une surface d'appui (49) de l'évidement cylindrique (47 ; 247 ; 347) avec profilage intégré.

3. Système selon la revendication 1 ou 2, **caractérisé en ce que** l'au moins une augmentation pédiculaire (241 ; 341A, B ; 441) et la vis pédiculaire (227 ; 327) s'étendent à travers une patte de liaison commune (51 ; 351), dans laquelle des passages axiaux (53, 55 ; 353A, B, 355) sont réalisés pour la réception par complémentarité de forme d'une section de tête (57 ; 357) de l'augmentation pédiculaire (241 ; 341A, B ; 441) et d'une section de guidage (229 ; 329 ; 429), située sous la tête de vis, de la vis pédiculaire (227 ; 327).

4. Système selon la revendication 3, **caractérisé en ce que** les passages axiaux sont formés par des passages circulaires cylindriques (53, 55 ; 353A, B, 355) se chevauchant.

5. Système selon la revendication 3 ou 4, **caractérisé en ce que** la patte de liaison (51 ; 351) sert à la fixation axiale de l'augmentation pédiculaire (241 ; 341A, B) et le cas échéant de butée axiale pour la vis pédiculaire (227 ; 327).

6. Système selon la revendication 5, **caractérisé en ce que** le passage axial (53 ; 353A, B) pour l'augmentation pédiculaire (241 ; 341A, B) porte une saillie de blocage (61) sur le côté opposé au passage (55 ; 355) pour la vis pédiculaire (227 ; 327), qui peut être reçue par complémentarité de forme dans une rainure de segment annulaire (59) de la section de tête (57) de l'augmentation pédiculaire (241 ; 341A, B).

7. Système selon la revendication 6, **caractérisé en ce que** la saillie de blocage (61) a une surface en saillie radiale depuis la paroi intérieure (53), qui est inférieure à la coupe transversale de l'évidement cylindrique (47) dans l'augmentation pédiculaire (241).

8. Système selon la revendication 5, **caractérisé en ce que** l'au moins une augmentation pédiculaire (341A, 341B) peut être matée avec la patte de liaison (351).

9. Système selon l'une quelconque des revendications 1 à 8, **caractérisé en ce que** l'évidement cylindrique (447) dans l'augmentation pédiculaire (441) est doté d'un filetage (71), lequel peut être mis en prise fonctionnelle avec la section filetée (429) de la vis pédiculaire (427).

10. Système selon la revendication 9, **caractérisé en ce que** le filetage (71) est contre-dépouillé dans l'augmentation pédiculaire (441).

11. Système selon l'une quelconque des revendications 1 à 10, **caractérisé en ce que** l'augmentation pédiculaire (41 ; 241 ; 341A, B ; 441) et/ou la patte de liaison (51 ; 351) sont en alliage de titane et présente de préférence au moins par zone un revêtement de titane poreux.

12. Kit pour un système d'ancrage d'une vis pédiculaire dans une vertèbre d'un patient, avec une vis pédiculaire (27 ; 127 ; 227 ; 327 ; 427) et une entretoise (35 ; 41 ; 241 ; 341A, B ; 441) selon l'une quelconque des revendications 1 à 11.

13. Kit selon la revendication 12, avec une patte de liaison (51 ; 351) selon l'une quelconque des revendications 3 à 8.

14. Entretoise pour un système d'ancrage d'une vis pédiculaire dans une vertèbre d'un patient, sous la forme d'une augmentation pédiculaire (41 ; 241 ; 341A, B ; 441) sensiblement cylindrique, qui a sensiblement la forme d'une vis d'ostéosynthèse ou d'une cheville osseuse, et un évidement cylindrique (47 ; 247 ; 347 ; 447) sur un côté de la surface circonférentielle extérieure, pour l'appui radial d'une section filetée (129 ; 229 ; 329 ; 429) d'une vis pédiculaire (127 ; 227 ; 327 ; 427).

15. Entretoise selon la revendication 14, avec une section de tête circulaire cylindrique (57), dans laquelle une rainure de segment annulaire (59) est réalisée pour la réception d'un élément de verrouillage (61) agissant axialement.

16. Entretoise selon la revendication 14, avec une section de tête (357), au niveau de laquelle des languettes rapportées (67) est réalisée pour le matage avec un élément de montage (351).

17. Entretoise selon l'une quelconque des revendications 14 à 16, **caractérisée en ce que** l'évidement cylindrique (447) est réalisé avec un filetage de préférence contre-dépouillé (71) pour la coopération avec une section filetée (73) d'une vis pédiculaire (427).
